# EUROPÄISCHE PATENTSCHRIFT

(11) **EP 3 054 003 B1**
(45) Veröffentlichungstag und Bekanntmachung des Hinweises auf die Patenterteilung: **14.08.2019**
(21) Anmeldenummer: 16154825.0
(22) Anmeldetag: 09.02.2016
(51) Int. Cl.: C12M 1/34, C12M 1/42, C12N 15/87, C12N 5/074, C12N 13/00

(54) **VERFAHREN UND VORRICHTUNG ZUR REPROGRAMMIERUNG VON LEBENDEN ZELLEN**
METHOD AND DEVICE FOR REPROGRAMMING OF LIVE CELLS
PROCEDE ET DISPOSITIF DE REPROGRAMMATION DE CELLULES VIVANTES

(30) Priorität: 09.02.2015 DE 102015101838
(43) Veröffentlichungstag der Anmeldung: 10.08.2016
(73) Patentinhaber: JenLab GmbH, 12559 Berlin (DE)
(72) Erfinder: KÖNIG, Karsten, 12559 Berlin (DE); KÖNIG, Aisada, 12559 Berlin (DE)
(74) Vertreter: Freitag, Joachim

(56) Entgegenhaltungen:
- WO-A1-2013/120960
- Aisada Uchugonova: "Optical reprogramming with ultrashort femtosecond laser pulses", Proc. SPIE, Conference Volume 9329, 7. Februar 2015 (2015-02-07), XP055261811, Gefunden im Internet: URL:http://proceedings.spiedigitallibrary. org/proceeding.aspx?articleid=2195731 [gefunden am 2016-03-31]
- Aisada Uchugonova: "Multiphoton Imaging and Nanoprocessing of Stem Cells with Near Infrared Femtosecond Laser Pulses", Dissertation - Medizinische Fakultät der Universität des Saarlandes, 26. Oktober 2009 (2009-10-26), XP055261746, Gefunden im Internet: URL:http://scidok.sulb.uni-saarland.de/vol ltexte/2010/3314/pdf/Uchugonova_PhDThesis_ Bibl.pdf [gefunden am 2016-03-31]
- AISADA UCHUGONOVA ET AL: "Optical reprogramming of human cells in an ultrashort femtosecond laser microfluidic transfection platform", JOURNAL OF BIOPHOTONICS, 4. November 2015 (2015-11-04), Seiten n/a-n/a, XP055261723, DE ISSN: 1864-063X, DOI: 10.1002/jbio.201500240

## Beschreibung

Die Erfindung betrifft ein Verfahren und eine Vorrichtung zur Reprogrammierung von lebenden Zellen, insbesondere zur virenfreien Reprogrammierung von adulten und embryonalen Stammzellen, iPS-Zellen sowie bereits differenzierten Zellen.

Der medizinische Einsatz von humanen adulten und embryonalen Stammzellen ist mit einer Reihe von Problemen verbunden. Adulte Stammzellen sind schwierig zu isolieren, verfügen oft nur über die Möglichkeit, in spezifische Gewebezellen zu differenzieren (multipotente Zellen), und sind bei der Entnahme vom Patienten oftmals geschädigt.

Embryonale humane Stammzellen sind dagegen pluripotent. Sie können sich in jeden Zelltyp differenzieren und können sich unter Beibehaltung der Pluripotenz selbst erneuern. Jedoch erfordert die Gewinnung dieser Zellen den Einsatz befruchteter humaner Eizellen. Der klinische Einsatz ist aufgrund bioethischer Gründe nur eingeschränkt möglich. Zudem müssen Immunsuppressiva verabreicht werden, da in der regenerativen Medizin beim Einsatz eines aus humanen embryonalen Stammzellen produzierten Transplantats die Gefahr der Abstoßung besteht.

Die genannten Nachteile des klinischen Einsatzes von Stammzellen treten beim Gebrauch von induziert pluripotenten Zellen (iPS) nicht auf. Solche iPS-Zellen entstehen durch Reprogrammierung von bereits differenzierten Zellen in undifferenzierte Zellen (Rückentwicklung), die sich ähnlich den embryonalen Stammzellen verhalten. Der genaue Mechanismus ist jedoch nicht bekannt. Im Jahre 2007 gelang die erste Herstellung von humanen iPS Zellen aus humanen Fibroblasten (Takahashi et al., Cell 131 (2007) 861-872).

Es bestehen verschiedene Methoden der Reprogrammierung. Viele Methoden, wie der Zellkern-Transfer oder die Zell-Transfusion, benötigen allerdings erneut den Einsatz von Eizellen.

Bei der direkten Reprogrammierung, bei der typischerweise drei oder vier spezielle Transkriptionsfaktoren oder Mikro-RNS in die differenzierte Zelle eingeschleust werden, besteht dieser Nachteil nicht. Bislang ging man davon aus, dass die Transkriptionsfaktoren mindestens für eine Woche stabil vorliegen müssen. Daher wurden diese in die genomische DNA eines Retrovirus oder Lenti-Virus integriert und appliziert [Miyazaki et al., Jpn. J. Clin. Oncol. 42 (2012) 773-779]. An diese Pioniere der viralen Reprogrammierung von Zellen wurde 2012 der Nobelpreis für Medizin und Physiologie vergeben.

Neben der Möglichkeit, zunächst iPS-Zellen durch Reprogrammierung herzustellen und anschließend für die medizinische Nutzung gezielt zu differenzieren, besteht auch die Möglichkeit einer teilweisen Reprogrammierung. In diesem Fall entstehen keine pluripotenten iPS-Zellen, sondern entweder direkt die gewünschten differenzierten Zellen, z. B. wird aus einer Hautzelle eine Herzzelle, oder multipotente Zellen, die sich nur in bestimmte Zellen differenzieren können [Efe et.al., Nature Cell Biology 13 (2011) 215-222,; Vierbuchen et.al., Nature 463 (2010) 7284]. Üblicherweise werden auch hier Viren verwendet, jedoch reichen oft drei Transkriptionsfaktoren aus. Von der teilweisen Reprogrammierung erhofft man sich zudem die Vermeidung von unkontrollierten Effekten, wie z. B. der Generierung von Tumorgewebe und unvorhersehbare Zellmutationen.

Der klinische Einsatz humaner reprogrammierter Zellen ist durch den Einsatz von Viren begrenzt bzw. derzeit nicht möglich. Zudem besteht der Nachteil der geringen Umwandlungseffizienz, d. h. zu wenige vitale Zellen gegenüber dem Anteil letaler Zellen nach einer viral injizierten Transkription.

Ein weiterer Nachteil ist es, dass das Verfahren der Herstellung von iPS-Zellen, die als dreidimensionale Cluster (iPS colony) vorliegen, langwierig ist und bislang mindestens eine Woche dauert.

Aus den iPS-Zellen oder den teilweise reprogrammierten Zellen werden in aufwendigen Schritten unter In-vitro-Bedingungen gewünschte Transplantate hergestellt. Zudem könnten iPS-Zellen direkt in ein Targetgewebe durch Injektion deponiert werden. Bislang werden lediglich embryonale und adulte Stammzellen direkt in das geschädigte Targetgewebe gespritzt (z. B. in den Herzmuskel, Rückenmark). Man erhofft sich dadurch effiziente Umwandlungen in den gewünschten differenzierten Zelltyp, da die Umgebung bereits aus diesen Zellen besteht und geeignete Differenzierungsfaktoren bereitstellt. Eine Reprogrammierung von Zellen innerhalb eines Gewebes ist derzeit nicht möglich. Von besonders großem Interesse wäre außerdem eine Reprogrammierung in situ innerhalb des zu behandelnden Gewebes im Patienten. Neben der Anwendung in der regenerativen Medizin wäre insbesondere die Reprogrammierung von Tumorstammzellen im Körper des Patienten von größtem Interesse.

Es bestehen daher folgende Problemkreise:
a) der Einsatz von Viren schränkt eine klinische Nutzung ein,
b) die Effizienz der Gewinnung von reprogrammierten Zellen ist gering,
c) die Herstellung reprogrammierter Zellen ist insgesamt zu langwierig,
d) die Herstellung reprogrammierter Zellen in einem Gewebeverband ist nicht möglich,
e) die Reprogrammierung von Zellen in krankem Targetgewebe eines Patienten ist auf viralem Wege unmöglich.

Derzeit werden verschiedene Methoden erforscht, um eine direkte Reprogrammierung ohne den Einsatz von Viren zu ermöglichen. So wurde durch die sogenannte STAP-Methode (Stimulus-triggered acquisition of pluripotency) einer japanischen Arbeitsgruppe, die auf der Variation der Mikroumgebung basiert, eine Reprogrammierung von Hautzellen der Maus realisiert und in der renommierten Zeitschrift Nature publiziert [Obokata et al., Nature 505 (2014) 641-647]. Die Methode wurde jedoch bislang nicht von anderen Forschungsgruppen verifiziert. Sie wurde bislang nur in tierischen Zellen appliziert. Zudem starben eine Vielzahl von Zellen, da die Mikroumgebung in Form von transienten extremen pH-Gradienten modifiziert wurde. Eine Reprogrammierung von Zellen in einem Gewebeverband ist mit dieser Methode nicht vorgesehen.

Bislang erfolgt die Reprogrammierung von Zellen üblicherweise durch den Einsatz von Viren, die eine klinische Anwendung behindern. Die neue STAP-Methode ist bislang auf tierische Zellen beschränkt und mit einer hohen Mortalitätsrate verbunden. Zudem erfolgen bislang alle Reprogrammierungen mit geringer Effizienz und in aufwendigen langwierigen Präparationsschritten. Eine direkte Reprogrammierung in einem In-vitro-Gewebe oder direkt in vivo am Patienten ist bislang nicht möglich.

Die Aufgabe der Erfindung besteht deshalb darin, eine neue Möglichkeit zur direkten Zell-Reprogrammierung zu finden, die auf den Prozessbedingungen der viralen Reprogrammierung basiert, aber ohne den Einsatz von Viren auskommt und eine effizientere und schnellere Reprogrammierung gestattet. Als erweiterte Aufgabenstellung soll eine direkte Reprogrammierung in vitalem Gewebe ermöglicht werden.

Es bestehen daher folgende Problemkreise:
a) der Einsatz von Viren schränkt eine klinische Nutzung ein,
b) die Effizienz der Gewinnung von reprogrammierten Zellen ist gering,
c) die Herstellung reprogrammierter Zellen ist insgesamt zu langwierig,
d) die Herstellung reprogrammierter Zellen in einem Gewebeverband ist nicht möglich,
e) die Reprogrammierung von Zellen in krankem Targetgewebe eines Patienten ist auf viralem Wege unmöglich.

Derzeit werden verschiedene Methoden erforscht, um eine direkte Reprogrammierung ohne den Einsatz von Viren zu ermöglichen. So wurde durch die sogenannte STAP-Methode (Stimulus-triggered acquisition of pluripotency) einer japanischen Arbeitsgruppe, die auf der Variation der Mikroumgebung basiert, eine Reprogrammierung von Hautzellen der Maus realisiert und in der renommierten Zeitschrift Nature publiziert [Obokata et al., Nature 505 (2014) 641-647]. Die Methode wurde jedoch bislang nicht von anderen Forschungsgruppen verifiziert. Sie wurde bislang nur in tierischen Zellen appliziert. Zudem starben eine Vielzahl von Zellen, da die Mikroumgebung in Form von transienten extremen pH-Gradienten modifiziert wurde. Eine Reprogrammierung von Zellen in einem Gewebeverband ist mit dieser Methode nicht vorgesehen.

Bislang erfolgt die Reprogrammierung von Zellen üblicherweise durch den Einsatz von Viren, die eine klinische Anwendung behindern. Die neue STAP-Methode ist bislang auf tierische Zellen beschränkt und mit einer hohen Mortalitätsrate verbunden. Zudem erfolgen bislang alle Reprogrammierungen mit geringer Effizienz und in aufwendigen langwierigen Präparationsschritten. Eine direkte Reprogrammierung in einem In-vitro-Gewebe oder direkt in vivo am Patienten ist bislang nicht möglich.

Die Aufgabe der Erfindung besteht deshalb darin, eine neue Möglichkeit zur direkten, effizienten und schnellen Zell-Reprogrammierung ohne den Einsatz von Viren und mit der Option einer direkten Reprogrammierung in vitalem Gewebe zu finden.

Erfindungsgemäß wird die Aufgabe mit einem Verfahren zur Reprogrammierung von lebenden Zellen, bei dem ein Cocktail aus mindestens zwei Transkriptionsfaktoren und einer Mikro-RNS ins Innere mindestens einer Zelle eingeschleust wird, um diese in iPS-Zellen oder einen anderen Zelltyp umzuwandeln, mit den folgenden Schritten gelöst:
- Bereitstellen eines Cocktails ohne viralen Träger mit mindestens drei herkömmlich für die Reprogrammierung verwendeten Transkriptionsfaktoren in einer wässrigen Umgebung der mindestens einen zu reprogrammierenden Zelle,
- Bereitstellen einer Strahlungsquelle in Form eines Femtosekunden-Lasers mit einer Impulswiederholfrequenz im Bereich zwischen 50 MHz und 2 GHz, einer Wellenlänge im Bereich von 700 bis 1200 nm,
- Richten eines Laserstrahls des Femtosekunden-Lasers mittels eines Laser-Scanning-Mikroskops mit einer numerischen Apertur zwischen 0,9 und 1,5 auf eine Zellmembran einer ausgewählten Zelle zu einem Fokus in einer Probe mit der mindestens einen Zelle auf einem verfahrbaren Kreuztisch,
- Richten eines abgeschwächten, nicht destruktiven Laserstrahls des Femtosekunden-Lasers, der auch für optische Bearbeitung verwendet wird, zur Einrichtung und Beobachtung der mindestens einen ausgewählten Zelle zum Fokus des Laser-Scanning-Mikroskops mittels eines Scanners und
- Steuern der Position der mindestens einen Zelle zum Fokus, der Belichtungsdauer und der Laserleistung für die optische Bearbeitung, sodass der Fokus in Abhängigkeit von der Impulsfolgefrequenz mit einer Leistung zwischen 7 mW und 100 mW eine transiente kleinporige Öffnung mit einer Größe im Bereich von bis zu 500 nm innerhalb einer Zellmembran der Zelle erzeugt, um eine Diffusion des Cocktails zur multiplen Reprogrammierung der Zelle durch die Zellmembran ins Innere der Zelle zu ermöglichen, sodass eine virenfreie optische multiple Reprogrammierung erfolgt.

Vorzugsweise erfolgt die Bestrahlung zur Reprogrammierung durch einen Femtosekunden-Laser mit einer Frequenz zwischen 75 und 85 MHz und einer Zentralwellenlänge zwischen 700 und 900 nm mittels des Laser-Scanning-Mikroskops über ein Mikroskopobjektiv mit einer hohen numerischen Apertur zwischen 1,1 und 1,3.

In einer bevorzugten Ausführung erfolgt die Bestrahlung zur Reprogrammierung mit einer Leistung zwischen 7 und 20 mW bei Impulslängen zwischen 5 und 20 fs und für eine Dauer zwischen 0,2 und 1 s.

Dabei ist es besonders zweckmäßig, die Bestrahlung zur Reprogrammierung mit einer Leistung zwischen 50 und 100 mW bei Impulslängen zwischen 100 und 200 fs und für eine Dauer zwischen 0,2 und 1 s durchzuführen.

Vorzugsweise werden zu reprogrammierende Zellen als Monolayer-Zellen auf einem Glassubstrat auf dem Kreuztisch bereitgestellt und mit dem virusfreien Cocktail in wässriger Lösung bedeckt, bevor die Bestrahlung zur Reprogrammierung beginnt.

In einer alternativen Variante des Verfahrens werden zu reprogrammierenden Zellen als wässrige Zellsuspension mit dem virusfreien Cocktail in einer mikrofluidischen Durchflusszelle durch eine Mikrokanüle geströmt.

Dabei erfolgt die Bestrahlung zur Reprogrammierung vorzugsweise mit einer Leistung zwischen 50 mW und 100 mW bei Verwendung eines in einem Besselstrahlmodus mit elongiertem Fokus geformten Laserstrahls, wobei der Laserstrahl einen elongierten Fokus über den gesamten Durchmesser der Mikrokanüle ausbildet und der Scanner des Laser-Scanning-Mikroskops orthogonal dazu einen Linienscan ausführt, um eine gesamte Querschnittsfläche der Mikrokanüle zu durchsetzen, und die Zellsuspension die Mikrokanüle mit einer Durchflussmenge von 135 bis 145 nl/s durchströmt.

Es ist dabei zweckmäßig, dass die Durchflusszelle in einem Kreislauf durchströmt wird, sodass die Zellsuspension aus zu programmierenden Zellen und dem virenfreien Cocktail die Mikrokanüle mehrfach (bis zu drei Wiederholungen) durchströmen kann, um mit dem gescannten elongierten Fokus die Trefferquote von zu reprogrammierenden Zellen zu erhöhen.

Vorteilhaft wird nach einer Diffusionszeit von mindestens fünf Sekunden nach Einwirkung der Bestrahlung der Plasmid-Cocktail um die reprogrammierten Zellen durch plasmidfreies Medium ausgetauscht und die Zellen werden im plasmidfreien Medium in einem Inkubator für mindestens zwei Tage bebrütet und gelagert.

Es erweist sich als zweckmäßig, wenn der Erfolg der optischen Reprogrammierung während der Lagerung im Inkubator mittels eines Fluoreszenzmikroskops durch Nachweis eines grün fluoreszierenden Proteins, das bei der Reprogrammierung während der transienten Öffnung der Zellmembran mit eingeschleust wurde, überwacht wird.

Vorteilhaft erfolgt mittels der optischen Bearbeitung eine optische multiple Reprogrammierung der mindestens einen Zelle in eine iPS-Zelle.

In einer weiteren vorteilhaften Ausführung kann mittels der optischen Bearbeitung der mindestens einen Zelle eine direkte optische Reprogrammierung durch Umwandlung eines Zelltypen in einen anderen Zelltypen erfolgen.

Des Weiteren kann die optische Bearbeitung in einem Gewebe aus einem dreidimensionalen Zellverbund als Perforation durch Bohren von Kanälen mit bis zu 10 µm Durchmesser genutzt werden.

Die Aufgabe wird weiterhin durch ein System zur Reprogrammierung von lebenden Zellen gelöst, umfassend einen virenfreien Cocktail mit mindestens drei Transkriptionsfaktoren aus herkömmlich für die Reprogrammierung verwendeten Plasmiden, der in wässriger Lösung um mindestens eine zu reprogrammierende Zelle angelagert ist, um in letztere eingeschleust zu werden und diese in iPS-Zellen oder einen anderen Zelltyp umzuwandeln, einen Femtosekunden-Laser zur gepulsten Bestrahlung der mindestens einen zu programmierbaren Zelle mit einer Frequenz zwischen 50 MHz und 2 GHz und einer Wellenlänge zwischen 700 und 1200 nm, um eine virenfreie optische Reprogrammierung zu erzeugen, ein Laser-Scanning-Mikroskop, das mit dem Femtosekunden-Laser ausgestattet ist und ein Mikroskopobjektiv mit einer hohen numerischen Apertur zwischen 0,9 und 1,5 aufweist, um den Laserstrahl des Femtosekunden-Lasers mit einem Fokus auf eine Zellmembran der mindestens einen zu reprogrammierenden Zelle zu richten, einen Kreuztisch als Träger zum Bereitstellen der mindestens einen zu reprogrammierenden Zelle in einer Zellsuspension des virenfreien Cockails mit den Transkriptionsfaktoren, der zu dem Mikroskopobjektiv des Laser-Scanning-Mikroskops so einrichtbar ist, dass fortlaufend zu reprogrammierende Zellen zur Bestrahlung bereitgestellt sind, und eine Steuereinheit zum Steuern der Position der mindestens einen zu reprogrammierenden Zelle zum Fokus des Laserstrahls, zur Leistungsumschaltung zwischen einem nichtdestruktiven Strahlungsmodus zur Beobachtung der zu programmierenden Zelle und einem Perforationsmodus und zum Steuern der Belichtungsdauer und der Laserleistung im Perforationsmodus, sodass im Fokus des Laserstrahls in Abhängigkeit von der Impulsfolgefrequenz eine Leistung zwischen 7 mW und 100 mW eingestellt ist und eine transiente kleinporige Öffnung mit einer Größe im Bereich von bis zu 500 nm innerhalb der Zellmembran der Zelle erzeugt wird, um wenigstens eine Perforierung innerhalb der Zellmembran der zu reprogrammierenden Zelle zu erreichen und eine Diffusion des virenfreien Cocktails durch die Zellmembran ins Innere der zu programmierenden Zelle zur multiplen optischen Reprogrammierung zu ermöglichen.

Vorzugsweise ist ein Kreuztisch vorhanden, mit dem die Positionierung von Monolayer-Zellen auf einem Glassubstrat zur Fokussierung eines Laserstrahls auf deren Zellmembran ausführbar ist.

Dabei ist der Femtosekunden-Laser zweckmäßig als fs-Laser mit einer Frequenz zwischen 75 und 85 MHz, einer Impulslänge zwischen 10 fs und 20 fs und einer Zentralwellenlänge zwischen 750 nm und 900 nm ausgebildet, der mittels des Mikroskopobjektivs mit einer hohen numerischen Apertur im Bereich von 1,1 bis 1,3 auf die Zellmembran mit einem Fokus im Submikrometerbereich bis zu 500 nm fokussierbar ist.

Besonders vorteilhaft weist der Kreuztisch eine mikrofluidische Durchflusszelle mit einer Mikrokanüle auf, die zur Durchströmung mit einer wässrigen Zellsuspension des Cocktails mit den zu reprogrammierenden Zellen vorgesehen ist.

Dabei ist der Femtosekunden-Laser vorzugsweise zur Aussendung eines Besselstrahls mit einem elongierten Fokus ausgebildet, wobei der Durchmesser der Mikrokanüle vollständig von einem mittels eines Scanners des Laser-Scanning-Mikroskops in einem Linienscan bewegten elongierten Fokus des Besselstrahls durchsetzt ist, sodass die zu reprogrammierenden Zellen den Fokus mit einer Strömungsgeschwindigkeit, die mit einer Durchflussmenge zwischen 135 und 145 nL/s bei einem Kanülendurchmesser von 100 µm erzeugt wird, durchqueren und während der Durchströmung getroffen werden.

Zweckmäßig ist nach der Durchflusszelle eine Zellkammer angeordnet, um die Zellsuspension aus reprogrammierten Zellen und Cocktail aufzufangen und den Cocktail durch ein plasmidfreies Medium zur Lagerung in einem Inkubator auszutauschen.

Die Erfindung basiert auf der Grundüberlegung, dass die Effizienz der Zell-Reprogrammierung entscheidend dadurch gesteigert werden kann, dass eine optische Reprogrammierung auf der Basis von ultrakurzen Laserpulsen zur Einschleusung der erforderlichen DNS-Plasmide und Mikro-RNS verwendet wird. Dabei wird eine transiente Änderung der Durchlässigkeit der Zellmembran erzeugt, indem diese durch fs-Laserimpulse perforiert wird und Transkriptionsfaktoren zur Reprogrammierung ins Innere der Zelle transportiert werden.

Für die erfindungsgemäße optische Zell-Reprogrammierung wird prinzipiell der Einsatz von ultrakurzen Laserimpulsen angewendet, wie er für laserbasierte dauerhafte Transfektion von DNS in lebende Zellen (gemäß US 7,892,837 B2) bekannt ist, wobei der Laser die Generierung von transienten Membranöffnungen bewirkt. Für diesen Zweck ist auch in der WO 2013/120960 A1 ein Durchfluss-Zytometer zur Femtosekunden-Laser-Perforation von Zellen beschrieben worden. Bislang wurde jedoch nur jeweils ein Gen mittels Lasereinwirkung eingeschleust, in der Regel ein Plasmid, das eine grüne Fluoreszenz hervorruft (sog. GFP-Protein). Die Reprogrammierung erfordert aber das Einschleusen multipler Gene bzw. Reprogrammierungsfaktoren (für die Herstellung von iPS-Zellen üblicherweise vier). Dafür ist die Verwendung von Lasern anstelle der problembehafteten viralen Reprogrammierung von Zellen ein völlig neuer Weg, der als "sterile optische Reprogrammierung" auch in räumlichen Zellverbänden und potenziell im menschlichen Körper risikoarme Reprogrammierungen von Zellen gestattet.

Eigene Vorarbeiten haben gezeigt, dass eine optische direkte, virenfreie Reprogrammierung, insbesondere die Herstellung humaner iPS-Zellen aus humanen Fibroblasten unter Verwendung von Femtosekunden-Lasern möglich ist. Dazu wurden die humanen Zellen in ein Medium, das die vier Transkriptionsfaktoren: Oct4, Nanog, Lin28 Sox2, sowie ein GFP-Plasmid (grünes fluoreszierendes Protein als Marker) enthielt, gegeben und mit zwölf Femtosekunden-Impulsen eines 85 MHz-Titan:Saphir-Lasers für wenige Millisekunden (50-100 ms) bestrahlt. Erstaunlicherweise reicht mitunter ein einzelner Beschuss der Zellen in einem speziellen Durchflusszytometer aus, mehrere iPS-Kolonien zu erzeugen, die infolge des zusätzlichen Einschleusens des GFP-Plasmids grün aufleuchteten. Interessanterweise erfolgte die Generation der iPS-Zell-Cluster (embryoic bodies) bereits nach 3-5 Tagen und damit wesentlich früher als bei viraler direkter Reprogrammierung. Überraschend ist neben der erreichbaren Schnelligkeit zudem die hohe Effizienz der optischen Reprogrammierung gegenüber der viralen Methode. So können einerseits iPS-Zellen hergestellt werden oder andererseits eine direkte Umwandlung eines Zelltypen in einen anderen Zelltypen, d.h. die sogenannte direkte Reprogrammierung, erfolgen, wie es im Stand der Technik bisher nur teilweise gelungen ist (siehe z. B. Efe et.al., Vierbuchen et.al. jeweils a.a.O., Szabo et al.: Direct conversion of human fibroblasts to multilieage blood progenitors, in: Nature 485 (2012) 585 oder Kim: Converting human skin cells to neurons: a new tool to study and treat brain disorders?, in: Cell Stem Cell 9 (2011) 179).

Prinzipiell kann das Lasersystem zur optischen Reprogrammierung auch direkt in dreidimensionalen Zellverbänden eingesetzt werden. Dazu müssen Transkriptionsfaktoren in die Mikroumgebung eingebracht werden. Dies kann z.B. durch eine mechanische Injektion erfolgen oder durch die erfindungsgemäße optische Herstellung von definierten Mikrokanälen von der Oberfläche des Gewebes zum Zielort im Gewebe mittels eines Femtosekundenlaser-Systems. Das Femtosekundenlaser-System soll dann auch genutzt werden, um die erforderlichen Membranporen zur Einschleusung der Transkriptionsfaktoren von der Mikroumgebung in die Zelle herzustellen. Dadurch kann Targetgewebe örtlich gezielt umprogrammiert werden. Dies erfolgt üblicherweise außerhalb des menschlichen Körpers, z.B. im Rahmen eines sog. Tissue Engineering. Prinzipiell kann das erfindungsgemäße Verfahren aber auch am Patienten eingesetzt werden.

Bisher medizinisch zugelassenen Femtosekunden-Lasersysteme, wie Multiphotonen-Tomographen für die Hautanalyse und Systeme für die Behandlung von Augenfehlsichtigkeit sind nicht geeignet, eine optische Reprogrammierung im Gewebe eines Patienten zu ermöglichen. Bei Tomographen sind die typischen 80 MHz-Laserimpulse mit einer Länge von 100 fs bis 200 fs und mittlerer Leistung von typischerweise 15 mW bei üblichen Strahlverweildauern von weniger als 100µs lediglich geeignet, um durch Zwei-Photonen-Fluoreszenz oder Frequenzverdoppelung (sog. SHG - Second Harmonic Generation) ausschließlich visuelle Bilddarstellung des Gewebes zu erzeugen. Dagegen beruhen Femtosekundenlaser für die Behandlung der Fehlsichtigkeit auf destruktiven, sogenannten photodisruptiven Effekten mittels energiereicher Femtosekunden-Laserimpulse im kHz-Bereich, wodurch plasmagefüllte Blasen und Schockwellen erzeugt werden, die Gewebestrukturen einschließlich des stabilen Kollagen-Netzwerks und ganzer Zellen zerstören. Dabei ist eine gezielte Einwirkung auf einzelne Zellen, um eine Bohrung eines transienten Kanals in die Membran der Zelle vorzunehmen, ohne diese irreversibel zu schädigen, definitiv nicht möglich.

Mit der erfindungsgemäßen optischen, virenfreien, vollständigen oder teilweisen Reprogrammierung von Zellen eröffnen sich vollkommen neue Therapie-Möglichkeiten, insbesondere auf dem Gebiet der regenerativen Medizin (z.B. der Herstellung von Transplantatgewebe) und der Behandlung von Krebserkrankungen.

Die Erfindung wird nachfolgend anhand von Ausführungsbeispielen und Abbildungen näher erläutert. Dabei zeigen:
- Fig. 1:: eine schematische Darstellung einer erfindungsgemäßen Anordnung zur Durchführung des Verfahrens;
- Fig. 2:: eine schematische Darstellung einer ersten Ausführungsform einer erfindungsgemäßen Vorrichtung zur Bearbeitung eines Monolayer-Zellverbunds;
- Fig. 3:: eine schematische Darstellung einer zweiten Ausführungsform einer erfindungsgemäßen Vorrichtung mit einer Fließzellen-Einrichtung, inklusive einer möglichen Mehrfachbearbeitung der durchfließenden Zellen;
- Fig. 4:: eine vergrößerte Detaildarstellung der Durchflusszelle gemäße Fig. 3 mit einer Darstellung eines gescannten Laserstrahls in Form eines Besselstrahls mit elongiertem Fokus.

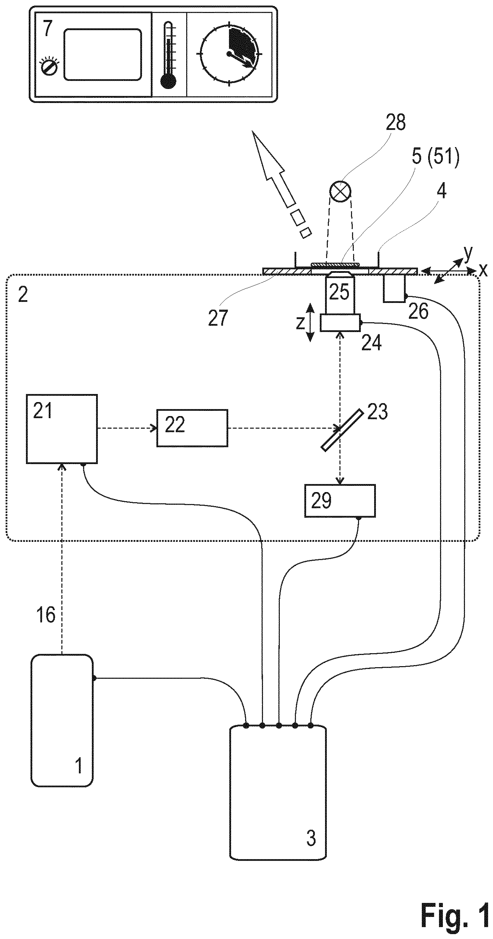

Der grundsätzliche Aufbau einer erfindungsgemäßen Vorrichtung gemäß Fig. 1 umfasst eine Strahlungsquelle 1, ein Laser-Scanning-Mikroskop 2 und eine Steuereinheit 3. Das Laser-Scanning-Mikroskop 2 verfügt über einen Kreuztisch 27, auf dem eine Probe 5 aufgenommen und in einer horizontalen x- und y-Richtung bewegt werden kann. Zur Beobachtung der Probe 5 durch ein Mikroskopobjektiv 25 verfügt das Laser-Scanning-Mikroskop 2 über eine Beleuchtung 28 und eine Videokamera 29. Die Bewegung des Kreuztisches 27 erfolgt durch einen Kreuztischantrieb 26. Die Bewegung des Mikroskopobjektivs 25 zur Fokussierung in einer vertikalen z-Richtung erfolgt mit einem Fokussierantrieb 24. Der Kreuztischantrieb 26 und der Fokussierantrieb 24 sind mit der Steuereinheit 3 verbunden.

Mit der Strahlungsquelle 1 wird ein gepulster Laserstrahl 16, mit dem im Fokus des Laser-Scanning-Mikroskops 2 Impulslängen im Bereich von 5 - 250 fs, bevorzugt im Bereich zwischen 5 - 200 fs, besonders bevorzugt zwischen 5 und 30 fs, und hoher Impulsfolgefrequenz im Bereich von 50 MHz bis 2 GHz, bevorzugt im Bereich zwischen 70 - 1000 MHz, bereitgestellt. In einer besonders bevorzugten Variante weist der Laserstrahl 16 Impulslängen von 10 - 20 fs und eine Impulsfolgefrequenz zwischen 75 und 85 MHz auf.

Mit dem Laserstrahl 16 werden Zellmembranen von in der Probe 5 befindlichen Zellen transient perforiert. Im Verlauf des Strahlengangs wird dazu der Laserstrahl 16 mittels eines Scanners 21 ausgelenkt und nach Durchlauf einer Strahlaufweitung 22 über einen Strahlteiler 23 in Richtung der Probe 5 gelenkt, wo er durch das Mikroskopobjektiv 25 mit sehr hoher numerischer Apertur zwischen 0,9 und 1,5, bevorzugt zwischen 1,1 und 1,3, in die Probe 5 fokussiert wird. Mit dem Scanner 21 wird der fokussierte Laserstrahl 16 über einen bestimmten Bereich der Probe 5 bewegt, sodass die Anzahl der mit dem fokussierten Laserstrahl 16 getroffenen Zellen erhöht werden kann. Der Strahlteiler 23 ermöglicht die Beobachtung der Probe 5 während der Verwendung des Laserstrahls 16. Die zeitliche und energetische Kontrolle des Laserstrahls 16 erfolgt ebenfalls mit der Steuereinheit 3.

Der Laserstrahl 16 wird gesteuert mit einem Shutter 12 zur zeitlichen Begrenzung der Lasereinwirkung an der Zellmembran bzw. für die Bohrung von Kanälen in einem Zellverbund (nachfolgend: Gewebe) und einer Steuereinheit 3 zur Leistungsumschaltung zwischen einem nichtdestruktiven Strahlungsmodus des Laserstrahls 16 zur Einstellung sowie Beobachtung der Zellen und einem Perforationsmodus zur transienten kleinporigen Öffnung der Zellmembran (Porengröße 10 nm bis 500 nm) zum Zwecke des Transfers (kurzzeitige Diffusion) von Mikro-RNS sowie Transkriptionsfaktoren (in Form von Plasmiden, episomal vectors, transposon).

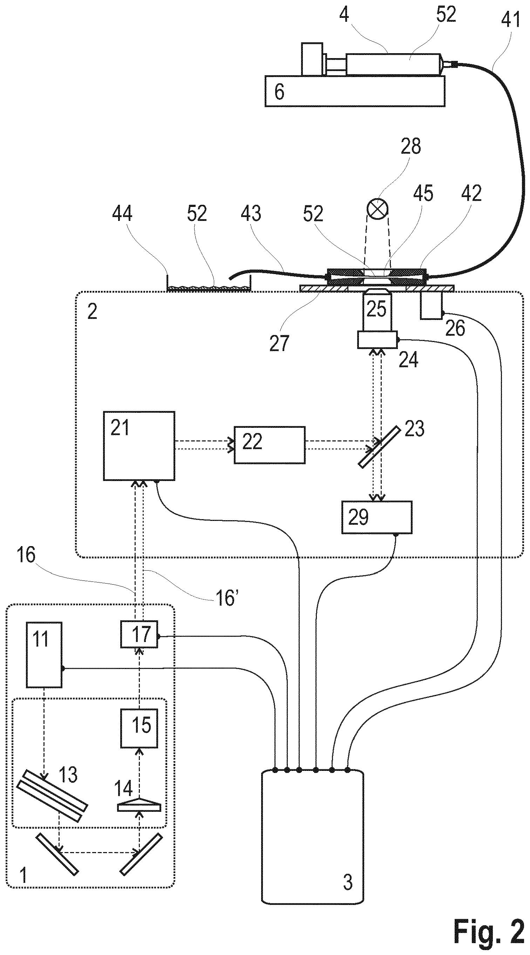

Zur Erreichung derart kleinporiger Öffnungen in der Zellmembran wird der Laserstrahl 16 vor Eintritt in das Laser-Scanning-Mikroskop 2 durch eine Strahlformungseinheit, die einen Dispersionskompensator 13, vorzugsweise in Form von gechirpten Spiegeln, ein Axikon 14 zur Erzeugung eines koaxialen Beleuchtungsringes und eine Periskopoptik 15 - wie beispielhaft in Fig. 2 dargestellt - enthalten kann, in seiner Lichtverteilung beeinflusst. Außerdem wird er durch eine Abschwächungseinheit 17 zur Steuerung der Ausgangsleistung des Lasers beeinflusst. Alle diese Einheiten verbessern die Homogenität bzw. die radiale Intensitätsverteilung in Richtung einer Anhebung der Randintensität des anfänglichen Gauß-Bündels für eine stärkere Fokussierung des Laserstrahls 16 im Zielvolumen der Probe 5 sowie seine Ausgangsleistung für den Betrachtungsmodus und für den Perforationsmodus.

### Ausführungsbeispiel 1

In einem ersten Ausführungsbeispiel wurden in einem Vorratsbehälter 4 humane Hautzellen der Firma LONZA (#CC-2511) als Monolayer-Zellen 51 angezüchtet. Für die Reprogrammierung der Zellen erfolgt die Zugabe einer die Monolayer-Zellen 51 bedeckenden Lösung in den Vorratsbehälter 4. Die Lösung enthält eine Plasmidmischung der Firma SBI (System Biosciences pMC-LGNSO MiniCircle DANN, #SRM100A-1) mit den Plasmiden Oct4, Lin28, Nanog, Sox2+GFP in einer Konzentration von 5-10 µg/ml.

Es wurden in einem Vorratsbehälter 4 mit einem Glasboden von 160 µm Dicke, die von den zu bearbeitenden Zellen einen Arbeitsabstand von 170 µm herstellt, humane Hautzellen der Firma Lonza (#CC-2511) als Monolayer-Zellen 51 angezüchtet. Den Monolayer-Zellen 51 wurde als Lösung eine Suspension in Form eines Plasmidcocktails der Firma SBI (System Biosciences), pMC-LGNSO MiniCircle DNA, #SRM100A-1, enthaltend die Plasmiden Oct 4, Lin 28, Nanog, Sox2 +GFP in einer Konzentration von 5-10 µg/ml, zugegeben, um den Schritt der Reprogrammierung biochemisch zu ermöglichen. Die Dicke des Glasbodens des Vorratsbehälters 4 entspricht ca. dem 40-fachen der numerischen Apertur des Mikroskopobjektivs 25, das in diesem Fall eine numerische Apertur von 1,3 aufwies.

Die so präparierten Zellen werden dann mittels eines Laser-Scanning-Mikroskops 2 der Bestrahlung eines Femtosekunden-Lasers 11 (10 fs, 85 MHz, Zentralwellenlänge 800 nm) mittels eines Mikroskopobjektivs 25 mit einer hohen numerischen Apertur von 1,3 ausgesetzt.

Prinzipiell kann der Femtosekunden-Laser 11 bevorzugt eine Impulsfolgefrequenz zwischen 80-85 MHz und Impulslängen zwischen 10-250 fs im Fokus aufweisen und kann mit Wellenlängen im Bereich zwischen 700 und 1200 nm verwenden. Wenn im Fokus des Laserstrahls 16 eine Impulslänge von 100 bis 200 fs verwendet wird, ist eine mittlere Leistung von 50-100 mW, bei Impulslängen zwischen 10 und 20 fs jedoch nur eine mittlere Leistung zwischen 7 und 15 mW einzustellen, um bei der Perforation der Zellmembran die einzelne Zelle nicht mortal zu schädigen.

Zum Auffinden einer geeigneten Membranposition einer ausgewählten Zelle wird ein motorisierter Kreuztisch 27 so verfahren, dass der Fokus des abgeschwächten, nichtdestruktiv wirkenden Laserstrahls 16 auf der Zellmembran positioniert wird.

Der abgeschwächte, nichtdestruktive Laserstrahl 16', der allein für die Bildgebung mittels Videokamera 29 erforderlich ist, wird dabei mit einer Leistung unter 5 mW betrieben.

Danach wird die mittlere Leistung des Laserstrahls 16 auf ca. 10-15 mW erhöht und die Membran für 50-100 ms bestrahlt. Dabei ist es auch möglich, eine einzelne Zelle an bis zu drei Positionen durch je eine Einzelbelichtung zu perforieren.

Die destruktive Wirkung durch Bohren einer transienten Öffnung mit einem Durchmesser im Bereich von 10 - 500 nm wird durch die Entstehung einer plasmagefüllten Kavitationsblase erreicht. Sie entsteht durch blitzartige Verdampfung des im Fokus befindlichen Volumens der Zellmembran und wird mittels Videokamera 29 registriert. Die maximal 5 µm große Kavitationsblase verschwand bei eigenen Experimenten nach ca. 5 Sekunden. In dieser Zeit konnte der Plasmidcocktail in die Zelle diffundieren.

Nach der Bestrahlung wird das Medium des Cocktails durch ein plasmidfreies Medium ausgewechselt und die Zellen im Inkubator 7 bei einer Gasatmosphäre aus 5% CO₂/95% Luft und bei 37°C gelagert. Die Aufnahme der Plasmide in die Zell-DNS kann anhand der Bildung des zugefügten grün fluoreszierenden GFP-Proteins (GFP - green fluorescence protein) mittels eines Fluoreszenzmikroskop nachgewiesen werden. Üblicherweise tritt die Grünfluoreszenz innerhalb von 12-36 Stunden nach Abschluss der Laserbestrahlung auf. Innerhalb der folgenden fünf Tage entstehen dreidimensionale grün fluoreszierende Zellcluster, deren Morphologie denen von viral erzeugten Zell-Clustern (embloic bodies) entspricht.

### Ausführungsbeispiel 2

Humane Hautzellen der Firma Lonze (#CC-2511) werden in einer Zellsuspension 52, die einen Plasmid-Cocktail der Firma SBI (System Biosciences), pMC-LGNSO MiniCircle DNA, #SRM100A-1, den Plasmiden Oct 4, Lin 28, Nanog, Sox2 +GFP enthält (3-4x höhere Konzentration als bei der Anwendung auf die Monolayer-Zellen 51 aus Beispiel 1), in einen Behälter einer Dosiereinrichtung 6 gefüllt.

Bei einer in Fig. 2 dargestellten Vorrichtung wird die Zellsuspension 52 aus der Dosiereinrichtung 6, die eine herkömmliche Spritze mit einem linearen Kolbenvorschub sein kann, mittels einer Zuleitung 41 einer Durchflusszelle 42 zugeführt. Die Durchflusszelle 42 enthält eine Mikrokanüle 45, die in diesem Beispiel einen Innendurchmesser von 100 µm aufweist und in der die Hautzellen nahezu vereinzelt werden, gestattet es der Zellsuspension 52, mit einer typischen Strömungsgeschwindigkeit von 18 µm/ms und einer Durchflussmenge von 139 nL/s durch die Mikrokanüle 45 hindurch zu strömen.

Ein Laserstrahl 16 des Femtosekunden-Lasers 11, dessen Strahlprofil mittels der Strahlformungseinheit in einen Quasi-Besselstrahl geformt wird, wobei die Strahlformungseinheit aus dem Dispersionskompensator 13, dem Axikon 14 und der Periskopoptik 15 besteht, weist nach dem Durchlaufen der Mikroskopoptik 25 eines Laser-Scanning-Mikroskops 2 einen elongierten Fokus über den gesamten Durchmesser der Mikrokanüle 45 auf und wird mit einer Folgefrequenz von 80 MHz permanent senkrecht zur Richtung der Mikrokanüle 45 eingestrahlt und dabei mittels des Scanners 21 orthogonal dazu in einem Linienscan mit 7-30 ms pro Linie bewegt, sodass er eine innere Querschnittsfläche der Mikrokanüle 45 permanent praktisch flächig durchsetzt und dadurch einen Großteil der zu reprogrammierenden Zellen (z. B. menschliche Hautzellen) innerhalb Mikrokanüle 45 der Durchflusszelle 42 perforiert. Die maximale mittlere Leistung des Laserstrahls 16 beträgt 135 mW in einem Quasi-Besselstrahl-Modus (bei Nutzung eines 10x, 0,13 NA Objektivs zur Fokussierung der Laserimpulse über die gesamte innere Querschnittsfläche der Mikrokanüle 45).

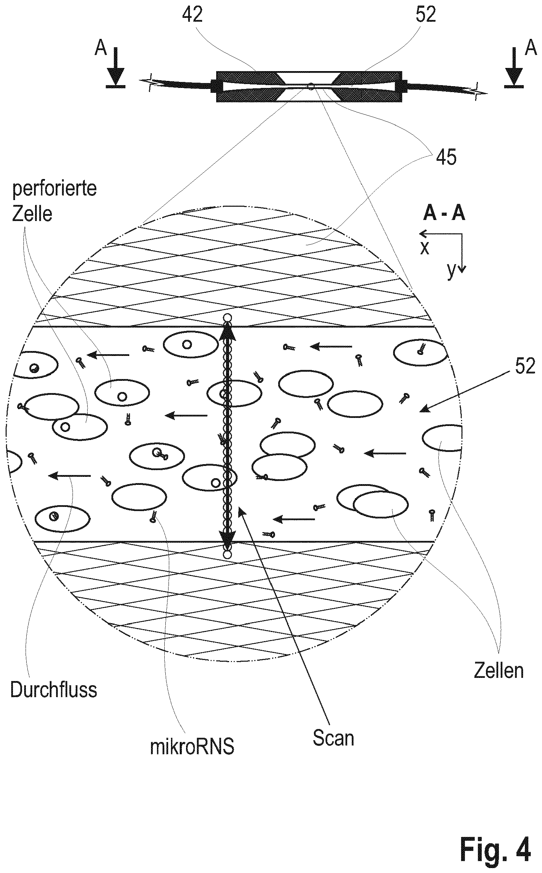

Fig. 4 zeigt zur Veranschaulichung der permanenten Durchsetzung der Mikrokanüle 45 mit dem gescannten elongierten Laserfokus bei durchströmender Zellsuspension 52 eine schematische Darstellung eine Schnittdarstellung der Mikrokanüle 45 in der Ebene A - A, wobei das Mikroskopobjektiv 52 (nicht gezeichnet) in dieser Darstellung von oben auf die Ebene A - A gerichtet ist. Der elongierte Fokus erstreckt sich senkrecht zur Zeichenebene und deckt den inneren Durchmesser der Mikrokanüle 45 in der Tiefe (in Z-Richtung ab). Die Y-Richtung wird durch den vom Scanner 21 des Laser-Scanning-Mikroskops 2 in der Vertikalen der Zeichnungsebene überstrichen und führt zu einer permanenten quasi-flächigen Ausbildung des Fokus über die gesamte innere Querschnittsfläche der Mikrokanüle 45. Dadurch wird bei fließender Zellsuspension 52 bereits eine hohe Effizienz der optischen Reprogrammierung durch laufende Perforierung der vorbeiströmenden Zellen erreicht.

Alle übrigen Einstellungen und Abläufe der Laserbestrahlung erfolgen in der gleichen Weise wie im Beispiel 1.

Die Zellen werden anschließend über eine Ableitung 43 aus der Mikrokanüle 45 über eine Ableitung 43 in eine standardisierte Zellkammer 44 abgeleitet und dort aufgefangen, dann gewaschen in einem Wachstumsmedium (growth medium) und mit dem Wachstumsmedium im Inkubator 7 bei 5%CO₂/95% Luft bei 37°C bebrütet/gelagert. Danach werden die infolge erfolgreicher Transfektion grün fluoreszierenden Zellen mittels Fluoreszenzmikroskop erfasst und durch Zentrifugieren abgetrennt, nachdem die übliche Zeitdauer von zwei bis fünf Tagen verstrichen ist.

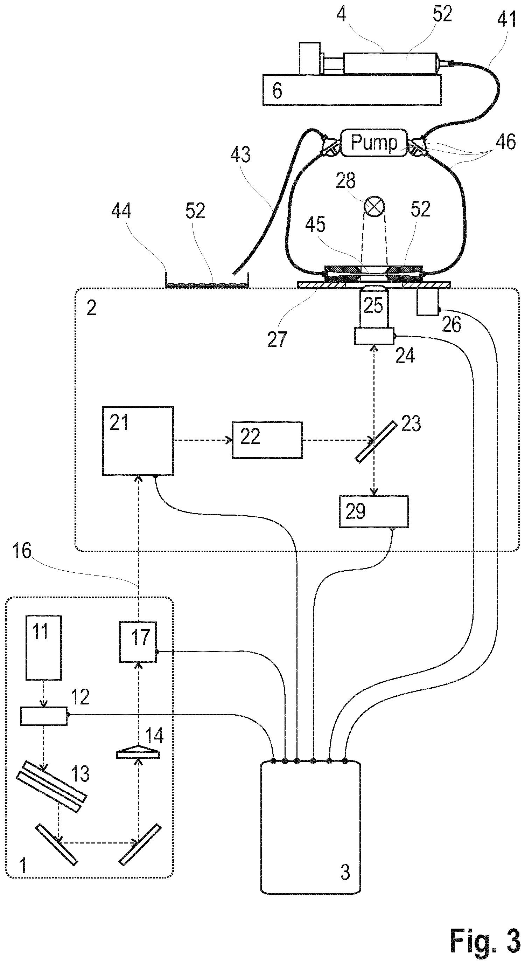

In einer modifizierten Vorrichtung gemäß Fig. 3, in der gegenüber Fig. 2 lediglich der Aufbau der der Durchflusszelle 42 abgewandelt ist, wird eine mehrfache Durchströmung der Mikrokanüle 45 mit der Zellsuspension 52 realisiert. Dazu wird ein Kreislaufsystem 46 aus Schläuchen an die Mikrokanüle 45 angeschlossen in dem eine Pumpe eingebaut ist, um das bereits einmal bestrahlte Zellsuspension 52 zwei- bis dreimal wiederholt durch die Mikrokanüle 45 zu strömen. Dadurch kann eine höhere Effizienz der Ausbeute an optisch multiple reprogrammierten Zellen erreicht werden. Da eine Zelle an ihrer Zellmembran auch mehrfach perforiert werden kann, ohne dass die Zelle mortal geschädigt wird, stellt auch eine mehrfach nacheinander erfolgende Perforation keine zusätzliche Gefährdung der zu reprogrammierenden Zellen dar. Alle übrigen Maßnahmen und Abläufe erfolgen in gleicher Weise wie zu Fig. 2 beschrieben.

### Bezugszeichenliste

- 1: Strahlungsquelle
- 11: Femtosekunden-Laser
- 12: Shutter
- 13: Dispersionskompensator (gechirpte Spiegel)
- 14: Axikon
- 15: Periskopoptik
- 16: Laserstrahl
- 16': abgeschwächter Laserstrahl
- 17: Abschwächungseinheit
- 2: Laser-Scanning-Mikroskop
- 21: Scanner
- 22: Strahlaufweitung
- 23: Strahlteiler
- 24: Fokussierantrieb (z-Richtung)
- 25: Mikroskopobjektiv
- 26: Kreuztischantrieb (x-y-Richtung)
- 27: Kreuztisch
- 28: Beleuchtung
- 29: Videokamera
- 3: Steuereinheit
- 4: Vorratsbehälter
- 41: Zuleitung
- 42: Durchflusszelle
- 43: Ableitung
- 44: Zellkammer
- 45: Mikrokanüle
- 46: Kreislaufsystem
- 5: Probe
- 51: Monolayer-Zellen
- 52: Zellsuspension
- 6: Dosiereinrichtung
- 7: Inkubator

## Patentansprüche

1. Verfahren zur Reprogrammierung von lebenden Zellen, bei dem ein Cocktail aus mindestens drei Transkriptionsfaktoren ins Innere mindestens einer Zelle eingeschleust wird, um diese in eine iPS-Zelle oder einen anderen Zelltyp umzuwandeln, umfassend die folgenden Schritte:
- Bereitstellen eines Cocktails ohne viralen Träger mit mindestens drei herkömmlich für die direkte Reprogrammierung verwendeten Transkriptionsfaktoren in einer wässrigen Umgebung der mindestens einen zu reprogrammierenden Zelle,
- Bereitstellen einer Strahlungsquelle (1) in Form eines Femtosekunden-Lasers (11) mit einer Impulswiederholfrequenz im Bereich zwischen 50 MHz und 2 GHz, einer Wellenlänge im Bereich von 700 bis 1200 nm,
- Richten eines Laserstrahls (16) des Femtosekunden-Lasers (11) mittels eines Laser-Scanning-Mikroskops (2) mit einer numerischen Apertur zwischen 0,9 und 1,5 auf eine Zellmembran der zu programmierenden Zelle zu einem Fokus in einer Probe (5) mit der mindestens einen Zelle auf einem verfahrbaren Kreuztisch (27),
- Richten eines abgeschwächten, nicht destruktiven Laserstrahls (16') des Femtosekunden-Lasers (11), der auch für optische Bearbeitung verwendet wird, zur Einrichtung und Beobachtung der mindestens einen ausgewählten Zelle zum Fokus des Laser-Scanning-Mikroskops (2) mittels eines Scanners (21) und
- Steuern der Position der mindestens einen Zelle zum Fokus, der Belichtungsdauer und der Laserleistung für die optische Bearbeitung, sodass der Fokus in Abhängigkeit von der Impulsfolgefrequenz mit einer Leistung zwischen 7 und 100 mW eine transiente kleinporige Öffnung mit einer Größe im Bereich von bis zu 500 nm innerhalb einer Zellmembran der Zelle erzeugt, um eine Diffusion des Cocktails zur multiplen Reprogrammierung der Zelle durch die Zellmembran ins Innere der Zelle zu ermöglichen, sodass eine virenfreie optische multiple Reprogrammierung erfolgt.

2. Verfahren nach Anspruch 1, wobei die Bestrahlung zur Reprogrammierung durch einen Femtosekunden-Laser (11) mit einer Frequenz zwischen 75 und 85 MHz und einer Zentralwellenlänge zwischen 700 und 900 nm mittels des Laser-Scanning-Mikroskops (2) über ein Mikroskopobjektiv (25) mit einer hohen numerischen Apertur zwischen 1,1 und 1,3 erfolgt.

3. Verfahren nach Anspruch 2, wobei die Bestrahlung zur Reprogrammierung mit einer Leistung zwischen 7 und 20 mW bei Impulslängen zwischen 5 und 20 fs und für eine Dauer zwischen 0,2 und 1 s erfolgt.

4. Verfahren nach Anspruch 2, wobei die Bestrahlung zur Reprogrammierung mit einer Leistung zwischen 50 und 100 mW bei Impulslängen zwischen 100 und 200 fs und für eine Dauer zwischen 0,2 und 1 s erfolgt.

5. Verfahren nach Anspruch 1, wobei zu reprogrammierende Zellen als Monolayer-Zellen (51) auf einem Glassubstrat auf dem Kreuztisch (27) bereitgestellt werden und mit dem virusfreien Cocktail bedeckt werden, bevor die Bestrahlung beginnt.

6. Verfahren nach Anspruch 1, wobei die zu reprogrammierenden Zellen als wässrige Zellsuspension (52) mit dem Cocktail durch eine mikrofluidische Durchflusszelle (42) mit einer Mikrokanüle (45) geströmt werden.

7. Verfahren nach Anspruch 6, wobei die Bestrahlung zur Reprogrammierung mit einer Leistung zwischen 50 und 100 mW bei Verwendung des in einem Besselstrahlmodus mit elongiertem Fokus geformten Laserstrahls (16) erfolgt, wobei der Besselstrahlmodus einen elongierten Fokus über den gesamten Durchmesser der Mikrokanüle (45) ausbildet und der Scanner (21) des Laser-Scanning-Mikroskops (2) orthogonal dazu einen Linienscan ausführt, um eine gesamte Querschnittsfläche der Mikrokanüle (45) zu durchsetzen, und die Zellsuspension (52) die Mikrokanüle (45) mit einer Durchflussmenge von 135 bis 145 nl/s durchströmt.

8. Verfahren nach Anspruch 6, wobei die Durchflusszelle (42) in einem Kreislauf durchströmt wird, sodass die Zellsuspension (52) aus zu programmierenden Zellen und dem virenfreien Cocktail die Mikrokanüle (45) mehrfach durchströmen kann, um mit dem gescannten elongierten Fokus die Trefferquote von zu reprogrammierenden Zellen zu erhöhen.

9. Verfahren nach einem der vorhergehenden Ansprüche, wobei nach einer Diffusionszeit von mindestens fünf Sekunden nach Einwirkung der Bestrahlung der Plasmid-Cocktail um die reprogrammierten Zellen durch plasmidfreies Medium ausgetauscht wird und die Zellen in einem Inkubator (7) für mindestens zwei Tage bebrütet und gelagert werden, wobei im Inkubator (7) der Erfolg der Reprogrammierung durch Nachweis des GFP-Proteins mittels eines Fluoreszenzmikroskops überwacht wird.

10. Verfahren nach Anspruch 1, wobei mittels der optischen Bearbeitung der mindestens einen Zelle entweder eine optische multiple Reprogrammierung in eine iPS-Zelle oder eine direkte optische Reprogrammierung durch Umwandlung eines Zelltypen in einen anderen Zelltypen erfolgt.

11. Verfahren nach Anspruch 1, wobei die optische Bearbeitung in einem Gewebe aus einem dreidimensionalen Zellverbund als Perforation durch Bohren von Kanälen mit bis zu 10 µm Durchmesser erfolgt.

12. System zur Reprogrammierung von lebenden Zellen, enthaltend
- einen Cocktail ohne viralen Träger mit mindestens drei herkömmlich für die direkte Reprogrammierung verwendeten Transkriptionsfaktoren, der in wässriger Lösung um mindestens eine zu reprogrammierende Zelle angelagert ist, um in letztere eingeschleust zu werden und diese in eine iPS-Zelle oder einen anderen Zelltyp umzuwandeln,
- einen Femtosekunden-Laser (11) zur gepulsten Bestrahlung der mindestens einen zu reprogrammierenden Zelle mit einer Frequenz zwischen 50 MHz und 2 GHz und einer Wellenlänge zwischen 700 und 1200 nm, um eine virenfreie optische Reprogrammierung der mindestens einen zu reprogrammierenden Zelle zu erzeugen,
- ein Laser-Scanning-Mikroskop (2), das mit dem Femtosekunden-Laser (11) ausgestattet ist und ein Mikroskopobjektiv (25) mit einer hohen numerischen Apertur zwischen 0,9 und 1,5 aufweist, um den Laserstrahl (16) des Femtosekunden-Lasers (11) mit einem Fokus auf eine Zellmembran der mindestens einen zu reprogrammierenden Zelle zu richten,
- einen Kreuztisch (27) als Träger zum Bereitstellen der mindestens einen zu reprogrammierenden Zelle in einer Zellsuspension (52) des virenfreien Cockails mit den Transkriptionsfaktoren, der zu dem Mikroskopobjektiv (25) des Laser-Scanning-Mikroskops (2) so einrichtbar ist, dass fortlaufend zu reprogrammierende Zellen zur fokussierten Bestrahlung der Zellmembran bereitgestellt sind, und
- eine Steuereinheit (3) zum Steuern der Position der mindestens einen zu reprogrammierenden Zelle zum Fokus des Laserstrahls (16), zur Leistungsumschaltung zwischen einem nichtdestruktiven Strahlungsmodus zur Beobachtung der zu programmierenden Zelle und einem Perforationsmodus und zum Steuern der Belichtungsdauer und der Laserleistung im Perforationsmodus, sodass im Fokus des Laserstrahls (16) in Abhängigkeit von der Impulsfolgefrequenz eine Leistung zwischen 7 mW und 100 mW eingestellt ist und eine transiente kleinporige Öffnung mit einer Größe im Bereich von bis zu 500 nm innerhalb der Zellmembran der Zelle erzeugt wird, um wenigstens eine Perforierung innerhalb der Zellmembran der mindestens einen zu reprogrammierenden Zelle zu erreichen und eine Diffusion des virenfreien Cocktails durch die Zellmembran ins Innere der zu reprogrammierenden Zelle zur multiplen optischen Reprogrammierung zu ermöglichen.

13. System nach Anspruch 12, wobei der Kreuztisch (27) zur Positionierung von Monolayer-Zellen (51) auf einem Glassubstrat und zur Fokussierung eines Laserstrahls (16) auf deren Zellmembran vorgesehen ist und der Femtosekunden-Laser (11) als Laser mit einer Frequenz zwischen 75 und 85 MHz, einer Impulslänge zwischen 10 und 20 fs und einer Zentralwellenlänge zwischen 750 und 900 nm ausgebildet ist, der mittels des Mikroskopobjektivs (25) mit einer hohen numerischen Apertur im Bereich zwischen 1,1 und 1,3 auf die Zellmembran mit einem Fokus im Submikrometerbereich zwischen 10 nm und 500 nm fokussierbar ist.

14. System nach Anspruch 12, wobei der Kreuztisch (27) eine mikrofluidische Durchflusszelle (42) mit einer Mikrokanüle (45) aufweist, die zur Durchströmung mit einer wässrigen Zellsuspension (52) des virenfreien Cocktails mit den zu reprogrammierenden Zellen vorgesehen ist, und der Femtosekunden-Laser (11) zur Aussendung eines Besselstrahls mit einem elongierten Fokus ausgebildet ist, und wobei der Durchmesser der Mikrokanüle (45) vollständig von einem mittels eines Scanners (21) des Laser-Scanning-Mikroskops (2) in einem Linienscan bewegten elongierten Fokus des Besselstrahls durchsetzt ist, sodass die zu reprogrammierenden Zellen den Fokus mit einer Strömungsgeschwindigkeit, die mit einer Durchflussmenge zwischen 135 und 145 nL/s bei einem Kanülendurchmesser von 100 µm erzeugt wird, durchqueren und während der Durchströmung getroffen werden.

15. System nach Anspruch 14, wobei nach der Durchflusszelle (42) eine Zellkammer (44) angeordnet ist, um die Zellsuspension (52) aus reprogrammierten Zellen und Cocktail aufzufangen und den Cocktail durch ein plasmidfreies Medium zur Lagerung in einem Inkubator (7) auszutauschen.

## Claims

1. Method for reprogramming living cells in which a cocktail comprising at least three transcription factors is transfected into the interior of at least one cell in order to convert this cell into an iPS cell or another type of cell, comprising the following steps:
- providing a cocktail without viral carrier with at least three transcription factors commonly used for direct reprogramming in an aqueous environment of the at least one cell to be reprogrammed,
- providing a radiation source (1) in the form of a femtosecond laser (11) with a pulse repetition frequency ranging between 50 MHz and 2 GHz with a wavelength ranging from 700 to 1200 nm,
- directing a laser beam (16) of the femtosecond laser (11) by means of a laser-scanning microscope (2) with a numerical aperture of between 0.9 and 1.5 on a cell membrane of the cell to be programmed in a focus in a sample (5) with the at least one cell on a displaceable x-y table (27),
- directing an attenuated, non-destructive laser beam (16') of the femtosecond laser (11), which is also used for optical treatment, for establishing and observing the at least one selected cell in the focus of the laser-scanning microscope (2) by means of a scanner (21), and
- controlling the position of the at least one cell with respect to the focus, the exposure period and laser output for the optical treatment such that the focus depending on the pulse repetition frequency with an output between 7 and 100 mW generates a transient small-pore opening with a size in the range of up to 500 nm within a cell membrane of the cell in order to allow a diffusion of the cocktail for multiple reprogramming of the cell through the cell membrane into the interior of the cell such that a virus-free optical multiple reprogramming takes place.

2. Method according to claim 1, wherein the irradiation for reprogramming by means of a femtosecond laser (11) is carried out at a frequency of between 75 and 85 MHz and with a center wavelength of between 700 and 900 nm by means of the laser-scanning microscope (2) via a microscope objective (25) with a high numerical aperture of between 1.1 and 1.3.

3. Method according to claim 2, wherein the irradiation for reprogramming is carried out at an output of between 7 and 20 mW with pulse lengths of between 5 and 20 fs and for a duration of between 0.2 and 1 seconds.

4. Method according to claim 2, wherein the irradiation for reprogramming is carried out at an output of between 50 and 100 mW with pulse lengths of between 100 and 200 fs and for a duration of between 0.2 and 1 seconds.

5. Method according to claim 1, wherein cells to be reprogrammed are provided as monolayer cells (51) on a glass substrate on the x-y table (27) and are covered with the virus-free cocktail before the start of irradiation.

6. Method according to claim 1, wherein the cells to be reprogrammed are streamed as aqueous cell suspension (52) with the cocktail through a microfluidic flow cell (42) with a micro-cannula (45).

7. Method according to claim 6, wherein the irradiation for reprogramming is carried out at an output of between 50 and 100 mW using the laser beam (16) shaped in a Bessel beam mode with elongated focus, wherein the Bessel beam mode forms an elongated focus over the entire diameter of the micro-cannula (45), and the scanner (21) of the laser-scanning microscope (2) carries out a line scan orthogonal thereto so as to interfuse an entire cross-sectional surface of the micro-cannula (45), and the cell suspension (52) flows through the micro-cannula (45) with a flow rate of 135 to 145 nl/s.

8. Method according to claim 6, wherein a flow is circulated through the flow cell (42) so that the cell suspension (52) of cells to be programmed and virus-free cocktail can flow through the micro-cannula (45) repeatedly in order to increase the hit ratio of cells to be reprogrammed with the scanned elongated focus.

9. Method according to one of the preceding claims, wherein after a diffusion time of at least five seconds after action of the irradiation, the plasmid cocktail around the reprogrammed cells is replaced by plasmid-free medium, and the cells are incubated and stored in an incubator (7) for at least two days, wherein the results of the reprogramming are monitored in the incubator (7) through detection of the GFP protein by means of a fluorescence microscope.

10. Method according to claim 1, wherein, by means of optical treatment of the at least one cell, either an optical multiple reprogramming into an iPS cell or a direct optical reprogramming through conversion of one cell type into another cell type is carried out.

11. Method according to claim 1, wherein the optical treatment is carried out in a tissue formed of a three-dimensional cell complex as perforation by boring channels with a diameter of up to 10 µm.

12. System for reprogramming living cells, containing
- a cocktail without viral carrier with at least three transcription factors commonly used for direct reprogramming which is located around at least one cell to be reprogrammed in an aqueous environment, in order to be transfected into the latter and to convert it into an iPS cell or another type of cell,
- a femtosecond laser (11) for pulsed irradiation of the at least one cell to be reprogrammed at a frequency of between 50 MHz and 2 GHz and a wavelength of between 700 and 1200 nm, in order to generate a virus-free optical reprogramming of the at least one cell to be reprogrammed,
- a laser-scanning microscope (2) which is outfitted with the femtosecond laser (11) and which has a microscope objective (25) with a high numerical aperture of between 0.9 and 1.5, in order to direct the laser beam (16) of the femtosecond laser (11) on a cell membrane of the at least one cell to be reprogrammed in a focus,
- an x-y table (27) as a carrier for providing the at least one cell to be reprogrammed in a cell suspension (52) of the virus-free cocktail with the transcription factors, which x-y table (27) can be arranged with respect to the microscope objective (25) of the laser-scanning microscope (2) such that cells to be reprogrammed are continuously provided for focused irradiation of the cell membrane, and
- a control unit (3) for controlling the position of the at least one cell to be reprogrammed with respect to the focus of the laser beam (16) for power switching between a non-destructive radiation mode for observation of the cell to be programmed and a perforation mode, and for controlling the exposure period and the laser output in the perforation mode, such that, depending on the pulse repetition frequency, an output of between 7 mW and 100 mW is set in the focus of the laser beam (16) and a transient small-pore opening with a size in the range of up to 500 nm is generated within the cell membrane of the cell, in order to achieve at least one perforation within the cell membrane of the at least one cell to be reprogrammed and to allow a diffusion of the virus-free cocktail through the cell membrane into the interior of the cell to be reprogrammed for multiple optical reprogramming.

13. System according to claim 12, wherein the x-y table (27) is provided for positioning monolayer cells (51) on a glass substrate and for focusing a laser beam (16) on the cell membrane of the monolayer cells (51), and the femtosecond laser (11) is configured as a laser with a frequency of between 75 and 85 MHz, a pulse length of between 10 and 20 fs and a center wavelength of between 750 and 900 nm and which can be focused on the cell membrane by means of the microscope objective (25) at a high numerical aperture in the range of between 1.1 and 1.3 in a focus in the submicrometer range of between 10 nm and 500 nm.

14. System according to claim 12, wherein the x-y table (27) has a microfluidic flow cell (42) with a micro-cannula (45) provided for flowing through an aqueous cell suspension (52) of the virus-free cocktail with the cells to be reprogrammed, and the femtosecond laser (11) is configured to emit a Bessel beam with an elongated focus, and wherein the diameter of the micro-cannula (45) is fully interfused by an elongated focus of the Bessel beam that is moved in a line scan by means of a scanner (21) of the laser-scanning microscope (2) such that the cells to be reprogrammed pass through the focus at a flow velocity generated by a flow rate of between 135 and 145 nL/s with a cannula diameter of 100 µm and are impinged while flowing through.

15. System according to claim 14, wherein a cell chamber (44) is arranged downstream of the flow cell (42) to capture the cell suspension (52) of reprogrammed cells and cocktail and for replacing the cocktail with a plasmid-free medium for storage in an incubator (7).

## Revendications

1. Procédé de reprogrammation de cellules vivantes, dans lequel un cocktail d'au moins trois facteurs de transcription est introduit à l'intérieur d'au moins une cellule pour la convertir en une cellule iPS ou en un autre type de cellule, comprenant les étapes suivantes:
- fournir un cocktail sans porteur viral avec au moins trois facteurs de transcription habituellement utilisés pour la reprogrammation directe dans un environnement aqueux de l'au moins une cellule à reprogrammer,
- fournir une source de rayonnement (1) sous la forme d'un laser femtoseconde (11) avec une fréquence de répétition des impulsions dans la plage d'entre 50 MHz et 2 GHz avec une longueur d'onde dans la plage de 700 à 1200 nm,
- diriger un faisceau laser (16) du laser femtoseconde (11) au moyen d'un microscope à balayage laser (2) avec une ouverture numérique d'entre 0,9 et 1,5 sur une membrane cellulaire de la cellule à programmer dans un foyer dans un échantillon (5) avec l'au moins une cellule sur un tableau croisé (27) mobile,
- diriger un faisceau laser (16') non destructif et affaibli du laser femtoseconde (11), qui est également utilisé pour le traitement optique, pour établir et observer l'au moins une cellule sélectionnée dans le foyer du microscope à balayage laser (2) au moyen d'un scanner (21) et
- contrôler la position de l'au moins une cellule par rapport au foyer, le temps d'exposition et la puissance laser pour le traitement optique, de sorte que le foyer, en fonction de la fréquence de répétition des impulsions avec une puissance d'entre 7 et 100 mW, génère une ouverture transitoire à petits pores d'une taille dans la plage allant jusqu'à 500 nm dans une membrane cellulaire de la cellule, pour permettre une diffusion du cocktail pour la reprogrammation multiple de la cellule à travers la membrane cellulaire jusqu'à l'intérieur de la cellule, de sorte qu'une reprogrammation multiple optique sans virus ait lieu.

2. Procédé selon la revendication 1, dans lequel l'irradiation pour la reprogrammation par un laser femtoseconde (11) est effectuée à une fréquence d'entre 75 et 85 MHz et une longueur d'onde centrale d'entre 700 et 900 nm au moyen du microscope à balayage laser (2) au moyen d'un objectif de microscope (25) avec une ouverture numérique élevée d'entre 1,1 et 1,3.

3. Procédé selon la revendication 2, dans lequel l'irradiation pour la reprogrammation est effectuée à une puissance d'entre 7 et 20 mW pour des longueurs d'impulsion d'entre 5 et 20 fs et pendant une durée d'entre 0,2 et 1 s.

4. Procédé selon la revendication 2, dans lequel l'irradiation pour la reprogrammation est effectuée à une puissance d'entre 50 et 100 mW pour des longueurs d'impulsion d'entre 100 et 200 fs et pendant une durée d'entre 0,2 et 1 s.

5. Procédé selon la revendication 1, dans lequel des cellules à reprogrammer sont fournies sous la forme de cellules monocouches (51) sur un substrat en verre sur un tableau croisé (27) et recouvertes du cocktail sans virus avant le début de l'irradiation.

6. Procédé selon la revendication 1, dans lequel les cellules à reprogrammer sous la forme d'une suspension cellulaire (52) aqueuse avec le cocktail passent par une cellule de débit (42) microfluidique avec une microcanule (45).

7. Procédé selon la revendication 6, dans lequel l'irradiation pour la reprogrammation est effectuée à une puissance d'entre 50 et 100 mW en utilisant le faisceau laser (16) mis en forme dans un mode de faisceau Bessel à foyer allongé, le mode de faisceau Bessel formant un foyer allongé sur tout le diamètre de la microcanule (45) et le scanner (21) du microscope à balayage laser (2) effectuant orthogonalement un balayage en ligne pour entremêler toute une section transversale de la microcanule (45), et la suspension cellulaire (52) traverse la microcanule (45) à une capacité de débit de 135 à 145 nl/s.

8. Procédé selon la revendication 6, dans lequel la cellule de débit (42) est traversée dans un circuit, de sorte que la suspension cellulaire (52) de cellules à programmer et du cocktail sans virus puisse traverser la microcanule (45) plusieurs fois pour augmenter le taux de réussite de cellules à reprogrammer avec le foyer allongé balayé.

9. Procédé selon une des revendications précédentes, dans lequel, après un temps de diffusion d'au moins cinq secondes après l'effet de l'irradiation, le cocktail plasmidique autour des cellules reprogrammées est remplacé par un milieu sans plasmide et les cellules sont incubées et stockées dans un incubateur (7) pendant au moins deux jours, le succès de la reprogrammation étant surveillé dans l'incubateur (7) par détection de la protéine GFP au moyen d'un microscope à fluorescence.

10. Procédé selon la revendication 1, dans lequel, au moyen du traitement optique de l'au moins une cellule, soit une reprogrammation multiple optique en une cellule iPS soit une reprogrammation optique directe en convertissant un type de cellule en un autre type de cellule est réalisée.

11. Procédé selon la revendication 1, dans lequel le traitement optique est réalisé dans un tissu d'un composite cellulaire tridimensionnel sous forme de perforation par perçage de canaux d'un diamètre allant jusqu'à 10 microns.

12. Système de reprogrammation de cellules vivantes, contenant
- un cocktail sans porteur viral avec au moins trois facteurs de transcription habituellement utilisés pour la reprogrammation directe qui est situé autour d'au moins une cellule à reprogrammer dans un environnement aqueux pour être introduit à l'intérieur de ladite cellule et pour la convertir en une cellule iPS ou en un autre type de cellule,
- un laser femtoseconde (11) pour l'irradiation pulsée de l'au moins une cellule à reprogrammer à une fréquence d'entre 50 MHz et 2 GHz et une longueur d'onde d'entre 700 et 1200 nm pour générer une reprogrammation optique sans virus de l'au moins une cellule à reprogrammer,
- un microscope à balayage laser (2) équipé du laser femtoseconde (11) et ayant un objectif de microscope (25) avec une ouverture numérique élevée d'entre 0,9 et 1,5 pour diriger le faisceau laser (16) du laser femtoseconde (11) sur une membrane cellulaire de l'au moins une cellule à reprogrammer dans un foyer,
- un tableau croisé (27) en tant que porteur pour fournir l'au moins une cellule à reprogrammer dans une suspension cellulaire (52) du cocktail sans virus avec les facteurs de transcription, le tableau croisé (27) pouvant être disposé par rapport à l'objectif de microscope (25) du microscope à balayage laser (2) de sorte que des cellules à reprogrammer puissent être fournies en continu pour l'irradiation focalisée de la membrane cellulaire, et
- une unité de contrôle pour contrôler la position de l'au moins une cellule à reprogrammer par rapport au foyer du faisceau laser (16) pour la commutation de puissance entre un mode de rayonnement non destructif pour observer la cellule à programmer et un mode de perforation et pour contrôler le temps d'exposition et la puissance laser dans le mode de perforation, de telle manière que, en fonction de la fréquence de répétition des impulsions, une puissance d'entre 7 mW et 100 mW est configurée dans le foyer du faisceau laser (16) et une ouverture transitoire à petits pores d'une taille allant jusqu'à 500 nm dans la membrane cellulaire de la cellule est générée pour obtenir au moins une perforation dans la membrane cellulaire de l'au moins une cellule à reprogrammer et pour permettre une diffusion du cocktail sans virus à travers la membrane cellulaire à l'intérieur de la cellule à reprogrammer pour la reprogrammation optique multiple.

13. Système selon la revendication 12, dans lequel le tableau croisé (27) servant à positionner des cellules monocouches (51) sur un substrat en verre et à focaliser un faisceau laser (16) est prévu sur la membrane cellulaire des cellules monocouches (51) et le laser femtoseconde (11) est conçu en tant que laser à une fréquence d'entre 75 et 85 MHz, une longueur d'impulsion d'entre 10 et 20 fs et une longueur d'onde centrale d'entre 750 et 900 nm qui peut être focalisé sur la membrane cellulaire au moyen de l'objectif de microscope (25) avec une ouverture numérique élevée dans la plage d'entre 1,1 et 1,3 dans un foyer dans la plage submicronique d'entre 10 nm et 500 nm.

14. Système selon la revendication 12, dans lequel le tableau croisé (27) a une cellule de débit (42) microfluidique avec une microcanule (45) destinée à s'écouler avec une suspension cellulaire (52) aqueuse du cocktail sans virus avec les cellules à reprogrammer, et le laser femtoseconde (11) est conçu pour émettre un faisceau Bessel avec un foyer allongé, et dans lequel le diamètre de la microcanule (45) est complètement entremêlé par un foyer allongé du faisceau Bessel déplacé dans un balayage linéaire au moyen d'un scanner (21) du microscope à balayage laser (2), de manière que les cellules à reprogrammer traversent le foyer avec une vitesse de débit qui est générée à une capacité de débit d'entre 135 et 145 nL/s à un diamètre de canule de 100 microns, et les cellules à reprogrammer sont touchées au cours du passage.

15. Système selon la revendication 14, dans lequel une chambre à cellules (44) est disposée en aval de la cellule de débit (42) pour capturer la suspension cellulaire (52) de cellules reprogrammées et cocktail et pour remplacer le cocktail par un médium sans plasmide pour le stockage dans un incubateur (7).
